# EUROPÄISCHE PATENTSCHRIFT

(11) **EP 0 794 974 B1**
(45) Veröffentlichungstag und Bekanntmachung des Hinweises auf die Patenterteilung: **02.02.2000**
(21) Anmeldenummer: 95940222.3
(22) Anmeldetag: 21.11.1995
(51) Int. Cl.: C08K 5/29, C08L 67/00

(54) **STABILISIERTE POLYESTER FORMMASSEN**
STABILIZED POLYESTER SHAPING COMPOUNDS
MATIERES DE MOULAGE EN POLYESTER STABILISEES

(30) Priorität: 01.12.1994 DE 4442724
(43) Veröffentlichungstag der Anmeldung: 17.09.1997
(73) Patentinhaber: BASF AKTIENGESELLSCHAFT, 67056 Ludwigshafen (DE)
(72) Erfinder: HEITZ, Thomas, D-67125 Dannstadt-Schauernheim (DE); HEYM, Manfred, D-67273 Weisenheim (DE); MÜHLBACH, Klaus, D-67269 Grünstadt (DE); PLACHETTA, Christoph, D-67117 Limburgerhof (DE)
(86) Internationale Anmeldenummer: EP9504577
(87) Internationale Veröffentlichungsnummer: WO9617011

(56) Entgegenhaltungen:
- EP-A- 0 628 541
- US-A- 5 357 021

## Beschreibung

Thermoplastische Formmassen, enthaltend
- A) 20 - 99 Gew.-% eines: Polyalkylenterephthalats mit 2 bis 10 C-Atomen im Alkohoiteil, welches gegebenenfalls bis zu 90 Gew.-% bezogen auf A) durch ein Polycarbonat oder ein Polyamid ersetzt sein kann
- B) 0,1 - 7 Gew.-%: eines Carbodiimids der Formel I 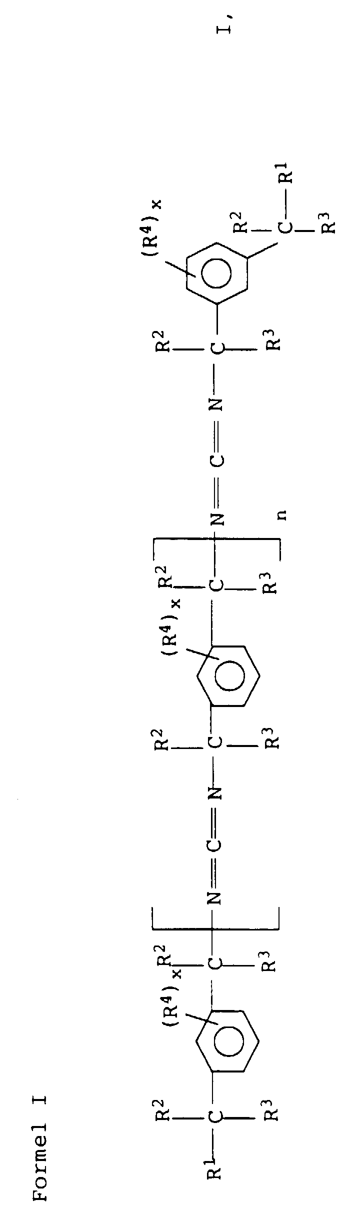 wobei
R¹ gleiche oder verschiedene Reste ausgewählt aus der Gruppe -NCO, -NHCONHR⁵, -NHCONR⁵R⁶ und -NHCOOR⁷ bedeutet, wobei
R⁵, R⁶ gleich oder verschieden sind und einen Alkyl-, Cycloalkyl-, oder Aralkylrest bedeuten,
R⁷ gleich R⁵ oder ein Alkoxy(poly)oxyalkylenrest ist und
R², R³ gleiche oder verschiedene aliphatische Reste mit 1 bis 18 C-Atomen oder cycloaliphatische Reste mit 5 bis 15 C-Atomen oder aromatische Reste mit 6 bis 15 C-Atomen bedeuten,
R⁴ gleiche oder verschiedene aliphatische Reste mit 2 bis 20 C-Atomen sowie Halogen oder ein Alkoxyrest
x eine ganze Zahl von 0 bis 4 und
n eine ganze Zahl von 0 bis 10 bedeutet,
- C) 0 bis 75 Gew.-%: üblicher Zusatzstoffe und Verarbeitungshilfsmittel,
wobei die Gewichtsprozente der Komponenten A) bis C) zusammen 100 % ergeben.

Außerdem betrifft die Erfindung die Verwendung der erfindungsgemäßen Formmassen zur Herstellung von Formkörpern jeglicher Art und die hierbei erhältlichen Formkörper.

Es ist bekannt, daß Polyester durch den Zusatz von phosphororganischen Verbindungen, phenolischen Antioxidantien oder auch aliphatischen bzw. aromatischen Aminen gegen Zerstörung durch Licht und Wärme geschützt werden können.

Die US 4,110,302 beschreibt glasfaserverstärkte Mischungen von PBT mit aromatischem Polycarbodiimid mit verbesserter Schlagzähigkeit. In Beispiel 1 wird Polytolylcarbodiimid verwendet.

US 4,071,503 offenbart Mischungen von PBT mit aromatischem Polycarbodiimid mit verbesserter Schmelzefestigkeit und Lösungsviskosität. Im angegebenen Beispiel wird Polytolylcarbodiimid verwendet.

DE-A 41 08 278 beschreibt mit mono- bzw. difunktionellen Carbodiimiden - im Beispiel auch mit kommerziell erhältlichem aromatischen Polycarbodiimid mit iso-Propylgruppen am Benzolring in ortho-Stellung zur Carbodiimidgruppe - stabilisiertes PBT zur Herstellung von Fasern mit verbesserter thermischer und hydrolytischer Beständigkeit.

BE-A 839 571 beschreibt mit aromatischem Polycarbodiimid modifiziertes PBT mit verbesserter Schmelzefestigkeit für Extrusions- und Blasformanwendungen. In der allgemeinen Beschreibung werden Polycarbodiimide beschrieben, die auf Tolyl-, Biphenyl- und Diphenylmethan basieren.

BE-A 846445 beschreibt faserverstärkte Mischungen gemäß BE-A 839,571 mit verbesserter Schlagzähigkeit für Extrusions- und Blasformanwendungen.

EP-A 898,213 beschreibt hydrolysestabile Blends aus Polyalkylterephthalat und Polycarbonat, die mit einem aromatischem Polycarbodiimid der Struktur stabilisiert sind:

Xₘ-[N=C=N-Y-]ₚ-(N=C=N-X)ₙ,

wobei X,Y = Aliphat oder Araliphat bedeutet, in dem mindestens eine ortho-Position zur CDI-Gruppe substituiert ist.

EP-A 46 04 81 offenbart PBT mit einem aromatischen Polycarbodiimid gemäß EP-A 598 213 der Struktur

Xₘ-[N=C=N-Y-]ₚ-(N=C=N-X)ₙ,

wobei X,Y = Aliphat oder Araliphat bedeutet, in dem mindestens eine ortho-Position zur CDI-Gruppe substituiert ist. Im angegebenen Beispiel wird das PCDI aus 1,3,5-Triisopropylbenzol-2,4-diisocyanat verwendet.

JP-A 61/014 251 beschreibt Mischungen von PBT und Copolymeren von Olefinen mit α,β-ungesättigten Säuren mit aromatischem Polycarbodiimid. In Beispiel 1 wird PET mit einem Zusatz von Poly(1,6-hexamethylen)carbodiimid verwendet.

EP-A 411 339 beschreibt Copolyester, die mit einer Stabilisatormischung aus einem Antioxidans auf Polychinolinbasis und einem aromatischem Polycarbodiimid mit verbesserter Alterungsbeständigkeit und Schmelzeviskosität. In Beispiel 1 wird Poly(1,4-phenylencarbodiimid) verwendet.

Aus dem Stand der Technik geht hervor, daß bei allen zur herstellung der beanspruchten Polycarbodiimide eingesetzten Diamine die Aminogruppe grundsätzlich direkt an einen Aromaten gebunden ist.

In der EP-A-628 541 werden neue Carbodiimide vorgeschlagen sowie deren Verwendung als Stabilisatoren für PolyureThane.

Ein Nachteil der mit den beschriebenen Polycarbodiimiden modifizierten Polyesterformmassen ist eine durch den hohen Aromatenanteil im Polycarbodiimid bedingte erhöhte Vergilbungstendenz. Durch die extreme sterische Hinderung der Aromaten an der Carbodiimidgruppierung ist bei deren Verarbeitung unter erhöhten Temperaturen eine Tendenz zur Isocyanatfreisetzung nicht zu unterbinden. Damit ist die Verarbeitung der beschriebenen Polycarbodiimide toxikologisch bedenklich. Die Hydrolysebeständigkeit - insbesondere gegenüber alkalischen Medien - der bekannten Formmassen ist darüber hinaus nicht zufriedenstellend.

Aufgabe der vorliegenden Erfindung war es daher, Polyesterformmassen mit besserer Hydrolysebeständigkeit, insbesondere gegenüber alkalischen Medien zu Verfügung zu stellen. Die Vergilbungsneigung sollte möglichst gering sein und bei der Verarbeitung die Formbelagsbildung (monomere Carbodiimide) sowie die Isocyanatentwicklung (oligomere Carbodiimide) weitestgehend minimiert werden.

Demgemäß wurden die eingangs definierten Formmassen gefunden. Bevorzugte Ausführungsformen sind den Unteransprüchen zu entnehmen.

Als Komponente (A) enthalten die erfindungsgemäßen Formmassen 20 bis 99, bevorzugt 35 bis 99 und Insbesondere 45 bis 98,5 Gew.-% eines thermoplastischen Polyalkylenterephthalates mit 2 bis 10 C-Atomen im Alkoholtell.

Derartige Polyalkylenterephthalate sind an sich bekannt und in der Literatur beschrieben. Sie enthalten einen aromatischen Ring in der Hauptkette, der von der aromatischen Dicarbonsäure stammt. Der aromatische Ring kann auch substituiert sein, z.B. durch Halogen wie Chlor und Brom oder durch C₁-C₄-Alkylgruppen wie Methyl-, Ethyl-, i- bzw. n-Propyl- und n-, i- bzw. t-Butylgruppen.

Diese Polyalkylenterephthalate können durch Umsetzung von aromatischen Dicarbonsäuren, deren Estern oder anderen esterbildenden Derivaten mit aliphatischen Dihydroxyverbindungen in an sich bekannter Weise hergestellt werden.

Als bevorzugte Dicarbonsäuren sind 2,6-Naphthalindicarbonsäure, Terephthalsäure und Isophthalsäure oder deren Mischungen zu nennen. Bis zu 30 mol-%, vorzugsweise nicht mehr als 10 mol-% der aromatischen Dicarbonsäuren können durch aliphatische oder cycloaliphatische Dicarbonsäuren wie Adipinsäure, Azelainsäure, Sebacinsäure, Dodecandisäuren und Cyclohexandicarbonsäuren ersetzt werden.

Von den aliphatischen Dihydroxyverbindungen werden Diole mit 2 bis 6 Kohlenstoffatomen, insbesondere 1,2-Ethandiol, 1,4-Butandiol, 1,6-Hexandiol, 1,4-Hexandiol, 1,4-Cyclohexandiol, 1,4-Cyclohexandimethylanol und Neopentylglykol oder deren Mischungen bevorzugt.

Als besonders bevorzugte Polyalkylenterephthalate (A) sind solche, die sich von Alkandiolen mit 2 bis 6 C-Atomen ableiten, zu nennen. Von diesen werden insbesondere Polyethylenterephthalat und Polybutylenterephthalat bevorzugt.

Die relative Viskosität der Polyalkylenterephthalate (A) liegt im allgemeinen im Bereich von 1,2 bis 1,8 (gemessen in einer 0,5 gew.-%igen Lösung in einem Phenol/o-Dichlorbenzolgemisch (Gew.-Verh. 1:1 bei 25°C).

Unter Polyalkylenterephthalaten im Sinne der vorliegenden Erfindung sollen auch Polycarbonate verstanden werden, die durch Polymerisation von aromatischen Dihydroxyverbindungen, insbesondere Bis-(4-hydroxyphenyl)2,2-propan (Bisphenol A) oder dessen Derivaten, z.B. mit Phosgen erhältlich sind. Entsprechende Produkte sind an sich bekannt und in der Literatur beschrieben sowie größtenteils auch im Handel erhältlich. Die Menge der Polycarbonate beträgt bis zu 90 Gew.-%, vorzugsweise bis zu 50 Gew.-%, bezogen auf 100 Gew.-% der Komponente (A).

Weiternin kann das Polyalkylenterephthalat A) durch bis zu 90 Gew.-%, vorzugsweise bis zu 50 Gew.-%, insbesondere bis zu 30 Gew.-% bezogen auf 100 Gew.-% A), durch ein Polyamid ersetzt werden. Es kommen sowohl teilkristalline als auch amorphe Polyamide in Betracht. Aufbaukomponenten für Polyamide und Verfahren zu deren Herstellung sind dem Fachmann bekannt, weshalb sich Einzelheiten hierzu erübrigen. Geeignete Polyamide sind beispielsweise unter dem Warenzeichen Ultramid® von der BASF AG im Handel erhältlich.

Als Komponente B) enthalten die erfindungsgemäßen Formmassen 0,1 bis 7, vorzugsweise 0,5 bis 5 und insbesondere 1,5 bis 3 Gew.-% eines Carbodiimids der Formel I 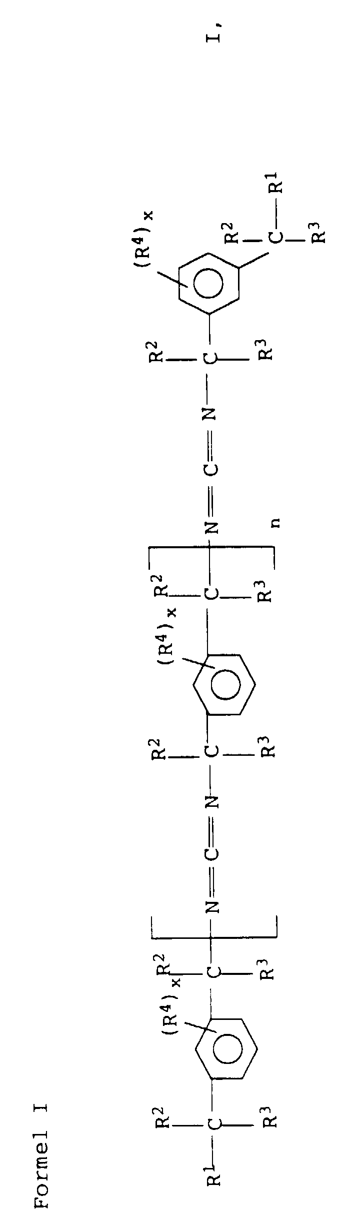 wobei R¹ gleiche oder verschiedene Reste ausgewählt aus der Gruppe -NCO, -NHCONHR⁵, NHCONR⁵R⁶ und -NHCOOR⁷ bedeutet, wobei
- R⁵, R⁶: gleich oder verschieden sind und einen Alkyl-, Cycloalkyl- oder Aralkylrest bedeuten,
- R⁷: gleich R⁵ oder ein Alkoxy(poly)oxyalkylenrest ist und
- R², R³: gleiche oder verschiedene aliphatische Reste mit 1 bis 18 C-Atomen oder cycloaliphatische Reste mit 5 bis 15 C-Atomen oder aromatische Reste mit 6 bis 15 C-Atomen bedeuten,
- R⁴: gleiche oder verschiedene aliphatische Reste mit 2 bis 20 C-Atomen sowie Halogen oder Alkoxyrest
- x: eine ganze Zahl von 0 bis 4 und
- n: eine ganze Zahl von 0 bis 10 bedeutet,

Bevorzugte aliphatische Reste R² und R³ sind Methyl, Isopropyl, 2-Methyl-propyl oder 2-Methylhexyl, wobei der Methylrest besonders bevorzugt ist.

Bevorzugte cycloaliphatische Reste R², R³ sind Cyclobutyl, Cyclohexyl oder Cycloundecyl, wobei Cyclohexyl besonders bevorzugt ist.

Als Beispiele für aromatische Reste R², R³ seien genannt: Phenyl, Naphthyl, 2-Tolyl oder Isopropylphenyl.

Als aliphatische Reste R⁴ kommen Methyl, Isopropyl oder 2-Methylpropyl in Betracht, wobei x für eine ganze Zahl von 0 bis 4, bevorzugt 0 oder 1 steht.

Bevorzugt sind Carbodiimide und/oder oligomere Polycarbodiimide der Formel (II) 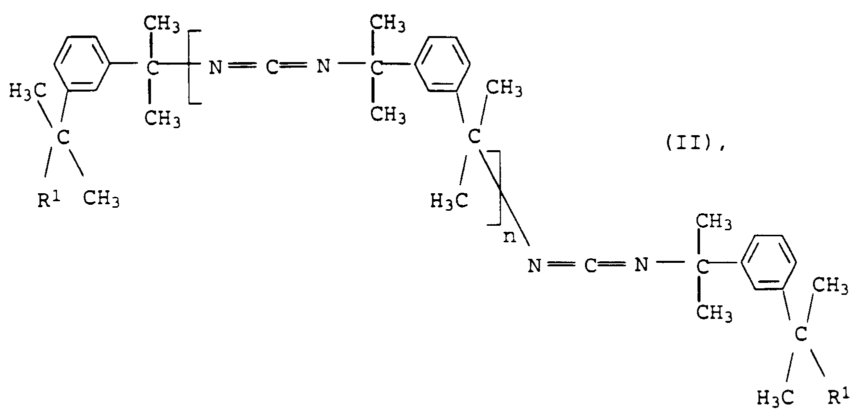 in der
- R¹: gleich oder verschieden und ausgewählt ist aus der Gruppe -NCO, -NHCONHR⁵, NHCONR⁵R⁶ und -NHCOOR⁷, wobei R⁵ und R⁶ gleich oder verschieden sind und einen Alkyl-, Cycloalkyl- oder Aralkylrest bedeuten und
- R⁷: gleich R⁵ oder ein Alkoxy(poly)oxyalkylenrest ist und
- n: eine ganze Zahl von 0 bis 10 ist.

Die erfindungsgemäßen Carbodiimide und oligomeren Polycarbodiimide besitzen an eine Methylengruppe gebundene, sterisch gehinderte Isocyanat-, Harnstoff- und/oder Urethangruppen, zeigen eine mit den technisch genutzten aromatischen Carbodiimiden und aromatischen Polycarbodiimiden zumindest vergleichbare hohe Hydrolyseschutzwirkung bei erhöhter Lichtbeständigkeit und können unter Beachtung der Arbeitsschutzvorschriften problemlos ohne zusätzliche Homogenisierungsschritte wirtschaftlich dosiert und in die Estergruppen enthaltenden Polykondensations- und Polyadditionsprodukte eingebracht werden. Vorteilhaft ist ferner die große Anzahl an wirksamen Carbodiimidgruppen, bezogen auf das Molekulargewicht der (Poly)carbodiimide, ihr geringer Dampfdruck und das vernachlässigbare Migrations- und Ausblühverhalten. Die (Poly)carbodiimide sind mit den Estergruppen enthaltenden Polyestern gut verträglich und aufgrund ihres niedrigen Schmelzpunktes auch problemlos mit diesen Materialien in der Schmelze homogen mischbar.

Aus den erfindungsgemäßen Carbodiimiden und oligomeren Polycarbodiimiden entstehen bei der Reaktion mit Carbonsäuren und/oder carboxylgruppenhaltigen Verbindungen araliphatische Isocyanate mit einer im Vergleich zu aromatischen Isocyanaten geringen Reaktivität. Folglich sind die Molekulargewichte der gebildeten Polyurethane und damit verbunden ihre mechanischen Eigenschaften konstant und sehr gut reproduzierbar. Vorteilhaft ist ferner, daß die aus den Isocyanaten entstehenden Abbauprodukte keine aromatischen Amingruppen gebunden haben und daher toxikologisch als relativ unproblematisch anzusehen sind.

Neben den monomeren Carbodiimiden finden als oligomere Polycarbodiimide vorteilhafterweise solche mit einem mittleren Kondensationsgrad (Zahlenmittelwert) von 2 bis 10, vorzugsweise von 2 bis 5 oder Mischungen davon oder Mischungen aus Mono- und oligomeren Polycarbodiimiden Verwendung, da diese sich in der Regel besonders gut in die zu stabilisierenden Estergruppen enthaltende Komponente A) einbringen lassen. Höherkondensierte Polycarbodiimide sind in der Regel feste, hochschmelzende Verbindungen, die mit der Kunststoffmatrix unzureichend verträglich und daher schwieriger mit den Polyadditions- oder Polykondensationsprodukten homogen mischbar sind.

Besonders bevorzugt sind Carbodiimide und/oder oligomere Polycarbodiimide der Formel (III), 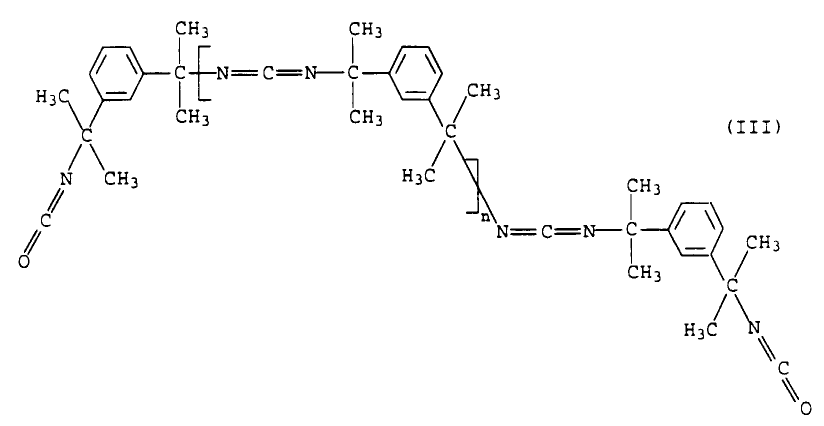 welche noch reaktive Isocyanatgruppen besitzen und daher mit Verbindungen mit NCO-reaktiven Wasserstoffatomen reagieren können und auf diese Weise in den Polyadditions- oder Polykondensationsprodukten chemisch gebunden werden. Zur Verbesserung der Lagerbeständigkeit der (Poly)carbodiimide können die endständigen Isocyanatgruppen beispielsweise mit C-H- oder N-H-reaktiven Verbindungen, wie z.B. Malonester, Acetylaceton, Acetessigester, Phthalimid, Caprolactam oder Benzolsulfonamid ganz oder teilweise verkappt oder durch Umsetzung mit aliphatischen, cycloaliphatischen oder araliphatischen Aminen, Alkoholen oder Polyoxyalkylenalkoholen teilweise oder vollständig abgesättigt und dadurch ihre physikalischen Eigenschaften, z.B. ihre Löslichkeit oder Verträglichkeit, gezielt modifiziert werden.

Zur Absättigung der Isocyanatgruppen der (Poly)carbodiimide können, wie bereits ausgeführt wurde, Amine, Alkohole und Polyoxyalkylenalkohole, verwendet werden. Geeignete Amine, z.B. primäre oder vorzugsweise sekundäre Amine, besitzen vorteilhafterweise 1 bis 12 C-Atome, vorzugsweise 2 bis 8 C-Atome. Beispielhaft genannt seien Methyl-, Ethyl-, Propyl-, Butyl-, Pentyl-, Hexyl-, 2-Ethylhexyl-, Octyl-, Decyl-, Diethyl-, Dipropyl-, Dibutyl-, Methylbutyl-, Ethylbutyl- und Ethylhexylamin sowie Cyclohexyl- und Benzylamin. Zur Absättigung der Isocyanatgruppen finden jedoch vorzugsweise Alkohole, z.B. primäre oder sekundäre Alkohole mit 1 bis 18 C-Atomen, vorzugsweise 2 bis 8 C-Atomen und insbesondere Alkoxypolyoxyalkylenalkohole mit 1 bis 10 C-Atomen, vorzugsweise 1 bis 4 C-Atomen, in der Alkoxygruppe und einem Molekulargewicht von 76 bis 2000, vorzugsweise 400 bis 1000 (Zahlenmittel) Verwendung. Als primäre oder sekundäre Alkohole seien beispielhaft genannt: Methanol, Ethanol, n-Propanol, Isopropanol, n-Butanol, sekundär-Butanol, n-Pentanol, technische Pentanolmischungen, n-Hexanol, technische Hexanolgemische, 2-Ethylhexanol, Octanol, 2-Ethyloctanol, Decanol und Dodecanol sowie Cyclohexanol und Benzylalkohol. Als Alkoxy(poly)oxyalkylenalkohole haben sich beispielsweise Polyoxybutylen-, Polyoxypropylen-, Polyoxypropylen-polyoxyethylen- und vorzugsweise Polyoxyethylenalkohole bewährt, die als endständige Alkoxygruppe, beispielsweise eine Methoxy-, Ethoxy-, n- oder iso-Propoxy- oder n-Butoxygruppe gebunden haben können. Je nach Art der verwendeten (Poly)oxyalkylenreste können die (Poly)carbodiimide hydrophil, wasserlöslich, bis hydrophob, fettlöslich, eingestellt werden.

Zur Herstellung der erfindungsgemäßen Carbodiimide und/oder oligomeren Polycarbodiimide kann 1,3-Bis-(1-methyl-l-isocyanatoethyl)-benzol bei erhöhten Temperaturen, z.B. bei Temperaturen von 50 bis 200°C, vorzugsweise von 150 bis 185°C, zweckmäßigerweise in Gegenwart von Katalysatoren unter Kohlendioxidabspaltung kondensiert werden. Hierfür geeignete Verfahren werden beispielsweise beschrieben in der GB-A-1 083 410, der DE-B 1 130 594 (GB-A-851 936) und der DE-A-11 56 401 (US-A-3 502 722). Als Katalysatoren vorzüglich bewährt haben sich z.B. Phosphorverbindungen, die vorzugsweise ausgewählt werden aus der Gruppe der Phospholene, Phospholenoxide, Phospholidine und Phospholinoxide. Wenn die Reaktionsmischung den gewünschten Gehalt an NCO-Gruppen, entsprechend einem Kondensationsgrad n bis 10 besitzt, wird die Polycarbodiimidbildung üblicherweise beendet. Hierzu können die Katalysatoren unter vermindertem Druck abdestilliert oder durch Zusatz eines Desaktivators, wie z.B. Phosphortrichlorid, desaktiviert werden. Die Polycarbodiimidherstellung kann ferner in Abwesenheit oder Gegenwart von unter den Reaktionsbedingungen inerten Lösungsmitteln durchgeführt werden.

Durch geeignete Wahl der Reaktionsbedingungen wie z.B. der Reaktionstemperatur, der Katalysatorart und der Katalysatormenge sowie der Reaktionszeit kann der Fachmann in der üblichen Weise den Kondensationsgrad einstellen. Der Verlauf der Reaktion kann am einfachsten durch Bestimmung des NCO-Gehaltes verfolgt werden. Auch andere Parameter wie z.B. Viskositätsanstieg, Farbvertiefung oder CO2-Entwicklung kann man für den Ablauf und die Steuerung der Reaktion heranziehen.

Nach beendeter Kondensation können, wie bereits ausgeführt wurde, die freien endständigen Isocyanatgruppen des Carbodiimids und/ oder der oligomeren Polycarbodiimide mit C-H- oder N-H-reaktiven Wasserstoffverbindungen blockiert oder mit aliphatischen, cycloaliphatischen und/oder araliphatischen Aminen, derartigen Alkoholen und/oder Alkoxypolyoxyalkylenalkoholen vollständig oder teilweise abgesättigt werden. Nach einer vorteilhaften Ausführungsform werden zur vollständigen Absättigung der Isocyanatgruppen die aliphatischen, cycloaliphatischen oder araliphatischen Amine, Alkohole und/oder Alkoxypolyoxyalkylenalkohole vorzugsweise in einem geringen Überschuß von -OH-, -NHR- und/oder -NH2-Gruppen zu NCO-Gruppen der (Poly)carbodiimide enthaltenden Reaktionsmischung hinzugefügt, dort ausreagieren gelassen und danach gegebenenfalls die nicht umgesetzte Menge, bevorzugt unter vermindertem Druck, abdestilliert werden.

Nach einer anderen, vorzugsweise angewandten Verfahrensvariante können die erfindungsgemäßen (Poly)carbodiimide mit teilweise oder vollständig abgesättigten Isocyanatgruppen in der Weise hergestellt werden, daß man zunächst bis zu 50 Gew.-%, vorzugsweise bis zu 23 Gew.-% der Isocyanatgruppen des 1,3-Bis-(1-methyl-1-isocyanato-ethyl)-benzols mit mindestens einem aliphatischen, cycloaliphatischen oder araliphatischen Amin, Alkohol und/oder Alkoxypolyoxyalkylenalkohol umsetzt und danach die freien Isocyanatgruppen in Gegenwart von Katalysatoren unter Kohlendioxidabspaltung ganz oder teilweise zu Carbodiimiden und/oder oligomeren Polycarbodiimiden kondensiert.

Die erfindungsgemäßen Monocarbodiimide und/oder oligomeren Polycarbodiimide eignen sich hervorragend als Akzeptor für Carboxylverbindungen und finden daher vorzugsweise Verwendung als Stabilisatoren gegen den hydrolytischen Abbau von Polyestern bzw. Polyester enthaltenden Blends.

Als Komponente C) können die erfindungsgemäßen Formmassen 0 bis 75, vorzugsweise bis zu 60 und insbesondere bis zu 50 Gew.-% üblicher Zusatzstoffe und Verarbeitungshilfsmittel enthalten.

Übliche Zusatzstoffe C) sind beispielsweise in Mengen bis zu 40, vorzugsweise bis zu 30 Gew.-% kautschukelastische Polymerisate (oft auch als Schlagzähmodifier, Elastomere oder Kautschuke bezeichnet).

Ganz allgemein handelt es sich dabei um Copolymerisate die bevorzugt aus mindestens zwei der folgenden Monomeren aufgebaut sind: Ethylen, Propylen, Butadien, Isobuten, Isopren, Chloropren, Vinylacetat, Styrol, Acrylnitril und Acryl- bzw. Methacrylsäureester mit 1 bis 18 C-Atomen in der Alkoholkomponente.

Derartige Polymere werden z.B. in Houben-Weyl, Methoden der organischen Chemie, Bd. 14/1 (Georg-Thieme-Verlag, Stuttgart, 1961). Seiten 392 bis 406 und in der Monographie von C.B. Bucknall, "Toughened Plastics" (Applied Science Publishers, London, 1977) beschrieben.

Im folgenden werden einige bevorzugte Arten solcher Elastomerer vorgestellt.

Bevorzugte Arten von solchen Elastomeren sind die sog. Ethylen-Propylen (EPM) bzw. Ethylen-Propylen-Dien-(EPDM)-Kautschuke.

EPM-Kautschuke haben im allgemeinen praktisch keine Doppelbindungen mehr, während EPDM-Kautschuke 1 bis 20 Doppelbindungen/100 C-Atome aufweisen können.

Als Dien-Monomere für EPDM-Kautschuke seien beispielsweise konjugierte Diene wie Isopren und Butadien, nicht-konjugierte Diene mit 5 bis 25 C-Atomen wie Penta-1,4-dien, Hexa-1,4-dien, Hexa-1,5-dien, 2,5-Dimethylhexa-1,5-dien und Octa-1,4-dien, cyclische Diene wie Cyclopentadien, Cyclohexadiene, Cyclooctadiene und Dicyclopentadien sowie Alkenylnorbornene wie 5-Ethyliden-2-norbornen, 5-Butyliden-2-norbornen, 2-Methallyl-5-norbornen, 2-Isopropenyl-5-norbornen und Tricyclodiene wie 3-Methyl-tricyclo(5.2.1.0.2.6)-3,8-decadien oder deren Mischungen genannt.

Bevorzugt werden Hexa-1,5-dien-5-ethyliden-norbornen und Dicyclopentadien. Der Diengehalt der EPDM-Kautschuke beträgt vorzugsweise 0,5 bis 50, insbesondere 1 bis 8 Gew.-%, bezogen auf das Gesamtgewicht des Kautschuks.

EPM- bzw. EPDM-Kautschuke können vorzugsweise auch mit reaktiven Carbonsäuren oder deren Derivaten gepfropft sein. Hier seien z.B. Acrylsäure, Methacrylsäure und deren Derivate, z.B. Glycidyl(meth)acrylat, sowie Maleinsäureanhydrid genannt.

Eine weitere Gruppe bevorzugter Kautschuke sind Copolymere des Ethylens mit Acrylsäure und/oder Methacrylsäure und/oder den Estern dieser Säuren. Zusätzlich können die Kautschuke noch Dicarbonsäuren wie Maleinsäure und Fumarsäure oder Derivate dieser Säuren, z.B. Ester und Anhydride, und/oder Epoxy-Gruppen enthaltende Monomere enthalten. Diese Dicarbonsäurederivate bzw. Epoxygruppen enthaltende Monomere werden vorzugsweise durch Zugabe von Dicarbonsäure- bzw. Epoxygruppen enthaltenden Monomeren der allgemeinen Formeln I oder II oder III oder IV zum Monomerengemisch in den Kautschuk eingebaut

R¹C (COOR²) =C (COOR³) R⁴ (I)

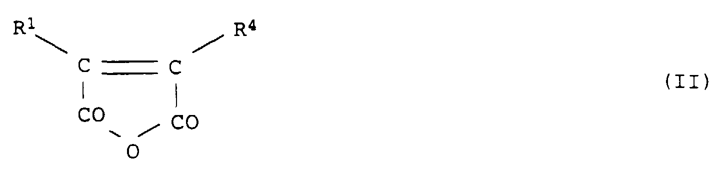

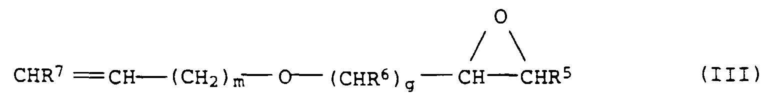

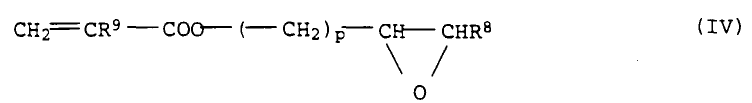

wobei R¹ bis R⁹ Wasserstoff oder Alkylgruppen mit 1 bis 6 C-Atomen darstellen und m eine ganze Zahl von 0 bis 20, g eine ganze Zahl von 0 bis 10 und p eine ganze Zahl von 0 bis 5 ist.

Vorzugsweise bedeuten die Reste R¹ bis R⁹ Wasserstoff, wobei m für 0 oder 1 und g für 1 steht. Die entsprechenden Verbindungen sind Maleinsäure, Fumarsäure, Maleinsäureanhydrid, Allylglycidylether und Vinylglycidylether.

Bevorzugte Verbindungen der Formeln I, II und IV sind Maleinsäure, Maleinsäureanhydrid und Epoxygruppen-enthaltende Ester der Acrylsäure und/oder Methacrylsäure, wie Glycidylacrylat, Glycidylmethacrylat und die Ester mit tertiären Alkoholen, wie t-Butylacrylat. Letztere weisen zwar keine freien Carboxylgruppen auf, kommen in ihrem Verhalten aber den freien Säuren nahe und werden deshalb als Monomere mit latenten Carboxylgruppen bezeichnet.

Vorteilhaft bestehen die Copolymeren aus 50 bis 98 Gew.-% Ethylen, 0,1 bis 20 Gew.-% Epoxygruppen enthaltenden Monomeren und/oder Methacrylsäure und/oder Säureanhydridgruppen enthaltenden Monomeren sowie der restlichen Menge an (Meth)acrylsäureestern.

Besonders bevorzugt sind Copolymerisate aus
- 50 bis 98,: insbesondere 55 bis 95 Gew.-% Ethylen,
- 0,1 bis 40,: insbesondere 0,3 bis 20 Gew.-% Glycidylacrylat und/oder Glycidylmethacrylat, (Meth)acrylsäure und/oder Maleinsäureanhydrid, und
- 1 bis 45,: insbesondere 10 bis 40 Gew.-% n-Butylacrylat und/oder 2-Ethylhexylacrylat.

Weitere bevorzugte Ester der Acryl- und/oder Methacrylsäure sind die Methyl-, Ethyl-, Propyl- und i- bzw. t-Butylester.

Daneben können auch Vinylester und Vinylether als Comonomere eingesetzt werden.

Die vorstehend beschriebenen Ethylencopolymeren können nach an sich bekannten Verfahren hergestellt werden, vorzugsweise durch statistische Copolymerisation unter hohem Druck und erhöhter Temperatur. Entsprechende Verfahren sind allgemein bekannt.

Bevorzugte Elastomere sind auch Emulsionspolymerisate, deren Herstellung z.B. bei Blackley in der Monographie "Emulsion Polymerization" beschrieben wird. Die verwendbaren Emulgatoren und Katalystoren sind an sich bekannt.

Grundsätzlich können homogen aufgebaute Elastomere oder aber solche mit einem Schalenaufbau eingesetzt werden. Der schalenartige Aufbau wird durch die Zugabereihenfolge der einzelnen Monomeren bestimmt; auch die Morphologie der Polymeren wird von dieser Zugabereihenfolge beeinflußt.

Nur stellvertretend seien hier als Monomere für die Herstellung des Kautschukteils der Elastomeren Acrylate wie z.B. n-Butylacrylat und 2-Ethylhexylacrylat, entsprechende Methacrylate, Butadien und Isopren sowie deren Mischungen genannt. Diese Monomeren können mit weiteren Monomeren wie z.B. Styrol, Acrylnitril, Vinylethern und weiteren Acrylaten oder Methacrylaten wie Methylmethacrylat, Methylacrylat, Ethylacrylat und Propylacrylat copolymerisiert werden.

Die Weich- oder Kautschukphase (mit einer Glasübergangstemperatur von unter 0°C) der Elastomeren kann den Kern, die äußere Hülle oder eine mittlere Schale (bei Elastomeren mit mehr als zweischaligem Aufbau) darstellen; bei mehrschaligen Elastomeren können auch mehrere Schalen aus einer Kautschukphase bestehen.

Sind neben der Kautschukphase noch eine oder mehrere Hartkomponenten (mit Glasübergangstemperaturen von mehr als 20°C) am Aufbau des Elastomeren beteiligt, so werden diese im allgemeinen durch Polymerisation von Styrol, Acrylnitril, Methacrylnitril, α-Methylstyrol, p-Methylstyrol, Acrylsäureestern und Methacrylsäureestern wie Methylacrylat, Ethylacrylat und Methylmethacrylat als Hauptmonomeren hergestellt. Daneben können auch hier geringere Anteile an weiteren Comonomeren eingesetzt werden.

In einigen Fällen hat es sich als vorteilhaft herausgestellt, Emulsionspolymerisate einzusetzen, die an der Oberfläche reaktive Gruppen aufweisen. Derartige Gruppen sind z.B. Epoxy-, Carboxyl-, latente Carboxyl-, Amino- oder Amidgruppen sowie funktionelle Gruppen, die durch Mitverwendung von Monomeren der allgemeinen Formel 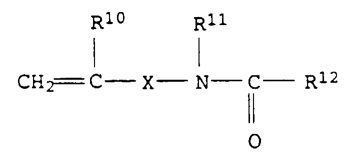 eingeführt werden können,
wobei die Substituenten folgende Bedeutung haben können:
- R¹⁰: Wasserstoff oder eine C₁- bis C₄-Alkylgruppe,
- R¹¹: Wasserstoff, eine C₁- bis C₈-Alkylgruppe oder eine Arylgruppe, insbesondere Phenyl,
- R¹²: Wasserstoff, eine C₁- bis C₁₀-Alkyl-, eine C₆- bis C₁₂-Arylgruppe oder -OR¹³
- R¹³: eine C₁- bis C₈-Alkyl- oder C₆- bis C₁₂-Arylgruppe, die gegebenenfalls mit O- oder N-haltigen Gruppen substituiert sein können,
- X: eine chemische Bindung, eine C₁- bis C₁₀-Alkylen- oder C₆-C₁₂-Arylengruppe oder 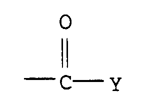
- Y: O-Z oder NH-Z und
- Z: eine C₁- bis C₁₀-Alkylen- oder C₆- bis C₁₂-Arylengruppe.

Auch die in der EP-A 208 187 beschriebenen Pfropfmonomeren sind zur Einführung reaktiver Gruppen an der Oberfläche geeignet.

Als weitere Beispiele seien noch Acrylamid, Methacrylamid und substituierte Ester der Acrylsäure oder Methacrylsäure wie (N-t-Butylamino)-ethylmethacrylat, (N,N-Dimethylamino)ethylacrylat, (N,N-Dimethylamino)-methylacrylat und (N,N-Diethylamino)ethylacrylat genannt.

Weiterhin können die Teilchen der Kautschukphase auch vernetzt sein. Als Vernetzer wirkende Monomere sind beispielsweise Buta-1,3-dien, Divinylbenzol, Diallylphthalat und Dihydrodicyclopentadienylacrylat sowie die in der EP-A 50 265 beschriebenen Verbindungen.

Ferner können auch sogenannten pfropfvernetzende Monomere (graftlinking monomers) verwendet werden, d.h. Monomere mit zwei oder mehr polymerisierbaren Doppelbindungen, die bei der Polymerisation mit unterschiedlichen Geschwindigkeiten reagieren. Vorzugsweise werden solche Verbindungen verwendet, in denen mindestens eine reaktive Gruppe mit etwa gleicher Geschwindigkeit wie die übrigen Monomeren polymerisiert, während die andere reaktive Gruppe (oder reaktive Gruppen) z.B. deutlich langsamer polymerisiert (polymerisieren). Die unterschiedlichen Polymerisationsgeschwindigkeiten bringen einen bestimmten Anteil an ungesättigten Doppelbindungen im Kautschuk mit sich. Wird anschließend auf einen solchen Kautschuk eine weitere Phase aufgepfropft, so reagieren die im Kautschuk vorhandenen Doppelbindungen zumindest teilweise mit den Pfropfmonomeren unter Ausbildung von chemischen Bindungen, d.h. die aufgepfropfte Phase ist zumindest teilweise über chemische Bindungen mit der Pfropfgrundlage verknüpft.

Beispiele für solche pfropfvernetzende Monomere sind Allylgruppen enthaltende Monomere, insbesondere Allylester von ethylenisch ungesättigten Carbonsäuren wie Allylacrylat, Allylmethacrylat, Diallylmaleat, Diallylfumarat, Diallylitaconat oder die entsprechenden Monoallylverbindungen dieser Dicarbonsäuren. Daneben gibt es eine Vielzahl weiterer geeigneter pfropfvernetzender Monomerer; für nähere Einzelheiten sei hier beispielsweise auf die US-PS 4 148 846 verwiesen.

Im allgemeinen beträgt der Anteil dieser vernetzenden Monomeren an dem schlagzäh modifizierenden Polymer bis zu 5 Gew.-%, vorzugsweise nicht mehr als 3 Gew.-%, bezogen auf das schlagzäh modifizierende Polymere.

Nachfolgend seien einige bevorzugte Emulsionspolymerisate aufgeführt. Zunächst sind hier Pfropfpolymerisate mit einem Kern und mindestens einer äußeren Schale zu nennen, die folgenden Aufbau haben:

| Typ | Monomere für den Kern | Monomere für die Hülle |
|---|---|---|
| I | Buta-1,3-dien, Isopren, n-Butylacrylat, Ethylhexylacrylat oder deren Mischungen | Styrol, Acrylnitril, Methylmethacrylat |
| II | wie I aber unter Mitverwendung von Vernetzern | wie I |
| III | wie I oder II | n-Butylacrylat, Ethylacrylat, Methylacrylat, Buta-1,3-dien, Isopren, Ethylhexylacrylat |
| IV | wie I oder II | wie I oder III aber unter Mitverwendung von Monomeren mit reaktiven Gruppen wie hierin beschrieben |
| V | Styrol, Acrylnitril, Methylmethacrylat oder deren Mischungen | erste Hülle aus Monomeren wie unter I und II für den Kern beschrieben zweite Hülle wie unter I oder IV für die Hülle beschrieben |

Anstelle von Pfropfpolymerisaten mit einem mehrschaligen Aufbau können auch homogene, d.h. einschalige Elastomere aus Buta-1,3-dien, Isopren und n-Butylacrylat oder deren Copolymeren eingesetzt werden. Auch diese Produkte können durch Mitverwendung von vernetzenden Monomeren oder Monomeren mit reaktiven Gruppen hergestellt werden.

Beispiele für bevorzugte Emulsionspolymerisate sind n-Butylacrylat/(Meth)acrylsäure-Copolymere, n-Butylacrylat/Glycidylacrylat- oder n-Butylacrylat/Glycidylmethacrylat-Copolymere, Pfropfpolymerisate mit einem inneren Kern aus n-Butylacrylat oder auf Butadienbasis und einer äußeren Hülle aus den vorstehend genannten Copolymeren und Copolymere von Ethylen mit Comonomeren, die reaktive Gruppen liefern.

Die beschriebenen Elastomere können auch nach anderen üblichen Verfahren, z.B. durch Suspensionspolymerisation, hergestellt werden.

Siliconkautschuke, wie in der DE-A 37 25 576, der EP-A 235 690, der DE-A 38 00 603 und der EP-A 319 290 beschrieben, sind ebenfalls bevorzugt.

Selbstverständlich können auch Mischungen der vorstehend aufgeführten Kautschuktypen eingesetzt werden.

Als faser- oder teilchenförmige Füllstoffe seien Kohlenstoffasern, Glasfasern, Glaskugeln, amorphe Kieselsäure, Asbest, Calciumsilicat, Calciummetasilicat, Magnesiumcarbonat, Kaolin, Kreide, gepulverter Quarz, Glimmer, Bariumsulfat und Feldspat genannt, die in Mengen bis zu 50 Gew.-%, insbesondere bis zu 40 % eingesetzt werden.

Als Komponente C) können die erfindungsgemäßen thermoplastischen Formmassen übliche Verarbeitungshilfsmittel wie Stabilisatoren, Oxidationsverzögerer, Mittel gegen Wärmezersetzung und Zersetzung durch ultraviolettes Licht, Gleit- und Entformungsmittel, Färbemittel wie Farbstoffe und Pigmente, Keimbildungsmittel, Weichmacher usw. enthalten.

Als Beispiele für Oxidationsverzögerer und Wärmestabilisatoren sind sterisch gehinderte Phenole, Hydrochinone, aromatische sekundäre Amine wie Diphenylamine, verschiedene substituierte Vertreter dieser Gruppen und deren Mischungen in Konzentrationen bis zu 1 Gew.-%, bezogen auf das Gewicht der thermoplastischen Formmassen genannt.

Als UV-Stabilisatoren, die im allgemeinen in Mengen bis zu 2 Gew.-%, bezogen auf die Formmasse, verwendet werden, seien verschiedene substituierte Resorcine, Salicylate, Benzotriazole und Benzophenone genannt.

Weiterhin können organische Farbstoffe wie Nigrosin, Pigmente wie Titandioxid, Cadmiumsulfid, Cadmiumselenid, Phthalocyanine, Ultramarineblau und Ruß als Farbstoffe zugesetzt werden.

Als Keimbildungsmittel können Natriumphenylphosphinat, Aluminiumoxid, Siliziumdioxid sowie bevorzugt Talkum eingesetzt werden.

Gleit- und Entformungsmittel, welche üblicherweise in Mengen bis zu 1 Gew.-% eingesetzt werden, sind bevorzugt langkettige Fettsäuren (z.B. Stearinsäure oder Behensäure), deren Salze (z.B. Ca- oder Zn-Stearat) oder Esterderivate (z.B. Stearylstearat oder Pentaerythrittetrastearat) sowie Amidderivate (z.B. Ethylen-bis-stearylamid).

Als Beispiele für Weichmacher seien Phthalsäuredioctylester, Phthalsäuredibenzylester, Phthalsäurebutylbenzylester, Kohlenwasserstofföle, N-(n-Butyl)benzolsulfonamid genannt.

Die erfindungsgemäßen Formmassen können noch 0 bis 2 Gew.-% fluorhaltige Ethylenpolymerisate enthalten. Hierbei handelt es sich um Polymerisate des Ethylens mit einem Fluorgehalt von 55 bis 76 Gew.-%, vorzugsweise 70 bis 76 Gew.-%.

Beispiele hierfür sind Polytetrafluorethylen (PTFE), Tetrafluorethylen-hexafluorethylen-Copolymere oder Tetrafluorethylen-Copolymerisate mit kleineren Anteilen (in der Regel bis zu 50 Gew.-%) copolymerisierbarer ethylenisch ungesättigter Monomerer. Diese werden z.B. von Schildknecht in "Vinyl and Related Polymers", Wiley-Verlag, 1952, Seite 484 bis 494 und von Wall in "Fluorpolymers" (Wiley Interscience, 1972) beschrieben.

Diese fluorhaltigen Ethylenpolymerisate liegen homogen verteilt in den Formmassen vor und weisen bevorzugt eine Teilchengröße d₅₀ (Zahlenmittelwert) im Bereich von 0,05 bis 10 µm, insbesondere von 0,1 bis 5 µm auf. Diese geringen Teilchengrößen lassen sich besonders bevorzugt durch Verwendung von wäßrigen Dispersionen von fluorhaltigen Ethylenpolymerisaten und deren Einarbeitung in eine Polyesterschmelze erzielen.

Zur besseren Verträglichkeit mit dem thermoplastischen Polyester sind Minerale und Füllstoffe gegebenenfalls mit einem Haftvermittler ausgerüstet. Bevorzugt sind Glycidyl-, Vinyl- und Aminoalkyltrialkoxysilane.

Als Flammschutzmittel seien organische Chlor- und Bromverbindungen, Erdalkalihydroxide, vorzugsweise mit Synergisten wie phosphororganischen Verbindungen und/oder Antimontrioxid sowie Mischungen von Carbonaten der II. Hauptgruppe und roter Phosphor genannt.

Die erfindungsgemäßen thermoplastischen Formmassen können nach an sich bekannten Verfahren hergestellt werden, in dem man die Ausgangskomponenten in üblichen Mischvorrichtungen wie Schneckenextrudern, Brabender-Mühlen oder Banbury-Mühlen mischt und anschließend extrudiert. Nach der Extrusion kann das Extrudat abgekühlt und zerkleinert werden. Es können auch einzelne Komponenten vorgemischt werden und dann die restlichen Ausgangsstoffe einzeln und/oder ebenfalls gemischt hinzugegeben werden. Die Mischtemperaturen liegen in der Regel bei 230 bis 290°C.

Nach einer bevorzugten Arbeitsweise können die Komponente B) sowie gegebenenfalls übliche Zusatzstoffe c) mit einem Polyesterpräpolymeren gemischt, konfektioniert und granuliert werden. Das erhaltene Granulat wird in fester Phase anschließend unter Inertgas kontinuierlich oder diskontinuierlich bei einer Temperatur unterhalb des Schmelzpunktes der Komponente A) bis zur gewünschten Viskosität kondensiert.

Die erfindungsgemäßen thermoplastischen Formmassen zeichnen sich durch eine gute Hydrolysebeständigkeit bei gleichzeitig guter Formstabilität aus. Sie eignen sich zur Herstellung von Fasern, Folien und Formkörpern, insbesondere für Anwendungen im Außenbereich oder Feuchtklima sowie Anwendungen mit Wasserkontakt.

### Beispiele

Es wurden folgende Komponenten eingesetzt:

Komponente A): Polybutylenterephthalat mit einer Viskositätszahl von 127 ml/g (Ultradur®B 4520 der BASF AG, VZ gemessen in 0,5 gew.-%iger Lösung aus Phenol/o-Dichlorbenzol, 1:1-Mischung bei 25°C).

Komponente B):

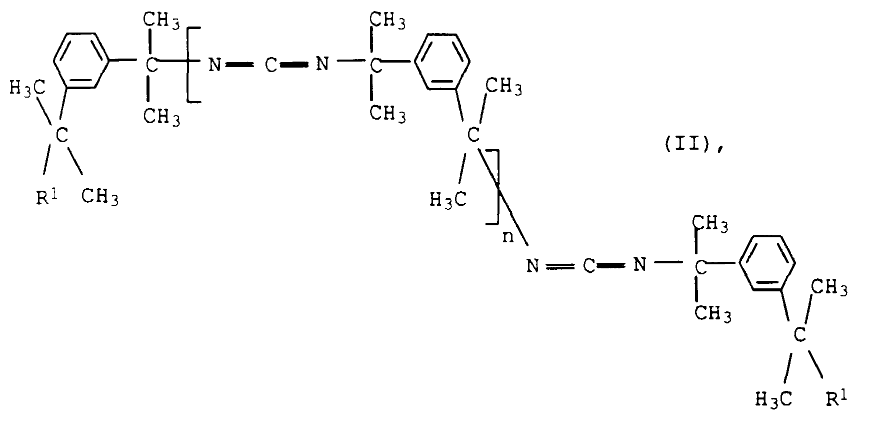

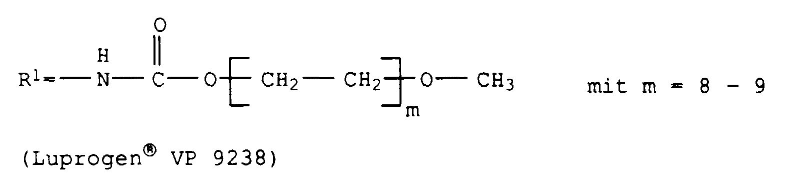

### Beispiele 1 bis 7 und Vergleichsbeispiel 1

Die Komponenten A) und B) wurden auf einem Zweischneckenextruder (ZSK 30, Werner & Pfleiderer; Drehzahl 200 Upm, Durchsatz 10 kg/h) bei 260°C abgemischt und in ein Wasserbad extrudiert. Nach Granulierung und Trocknung wurden auf einer Spritzgußmaschine Prüfkörper gespritzt und geprüft.

Die Lagerung der Formkörper für die Hydrolysetests erfolgte über die angegebene Zeit in Wasser bei einer Temperatur von 100°C im Autoklaven (Eigendruck).

Die mechanischen Eigenschaften wurden nach folgenden Methoden bestimmt:

Zugversuch nach ISO 527-2 (an Probekörper ISO 3167: 1993 Typ 1A) bei einer Dehnrate von 5 mm/min (Anfangsdehnrate von 1 mm/min zur Bestimmung des E-Moduls).

Schlagbiegeversuch nach ISO 179 am Probekörper leU bei 23°C (Charpy).

Kerbschlagversuch nach ISO 179 am Probekörper 1eA (S) mit gespritzter Kerbe bei 23°C.

Schmelzindex (MV) bei 250°C mit einer Aufschmelzzeit von 4 min und einer Auflagekraft von 2,16 kg.

Lösungsviskosität bei 25°C an einer 0,5 %igen Lösung von PBT in einer Mischung von ortho-Dichlorbenzol und Phenol (1:1).

Die Ergebnisse der Messungen und die Zusammensetzung der Formmassen sind der Tabelle 1 zu entnehmen.

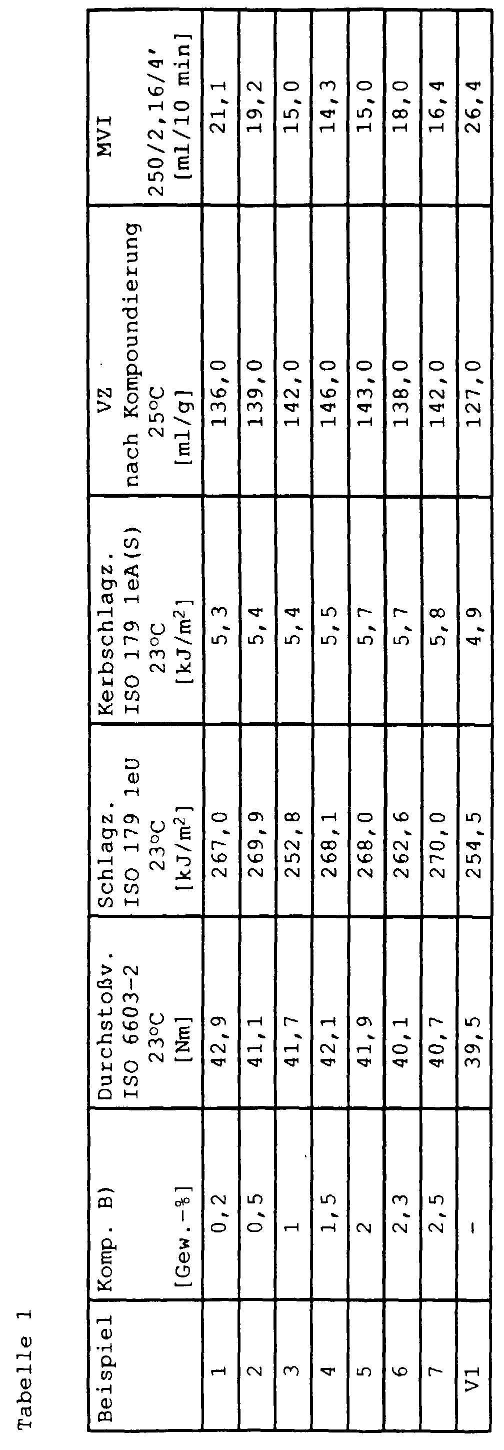

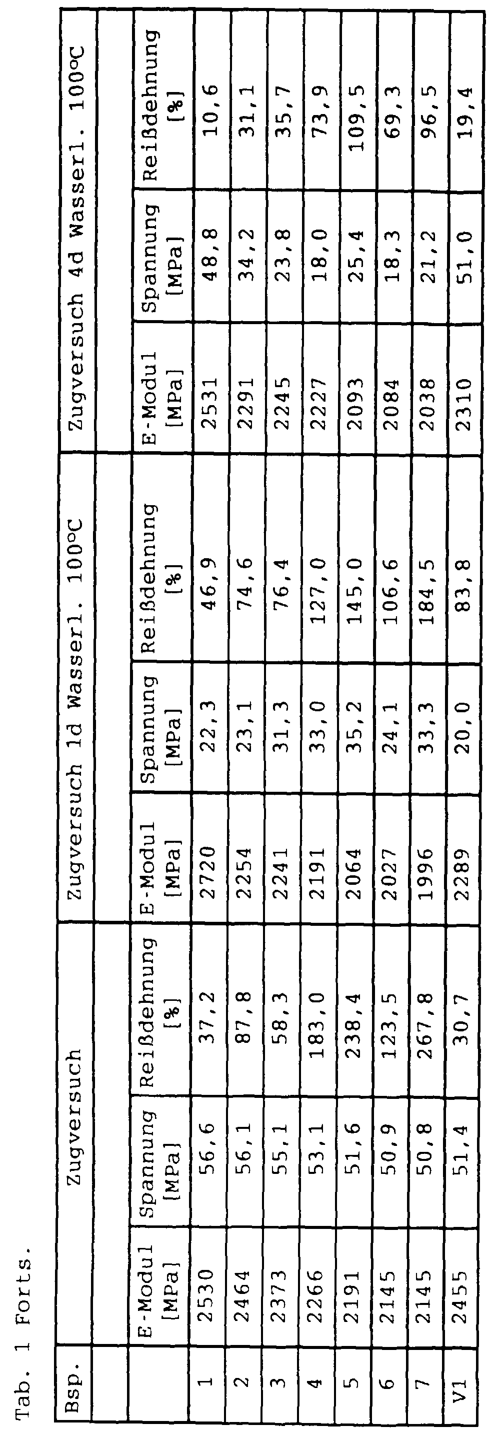

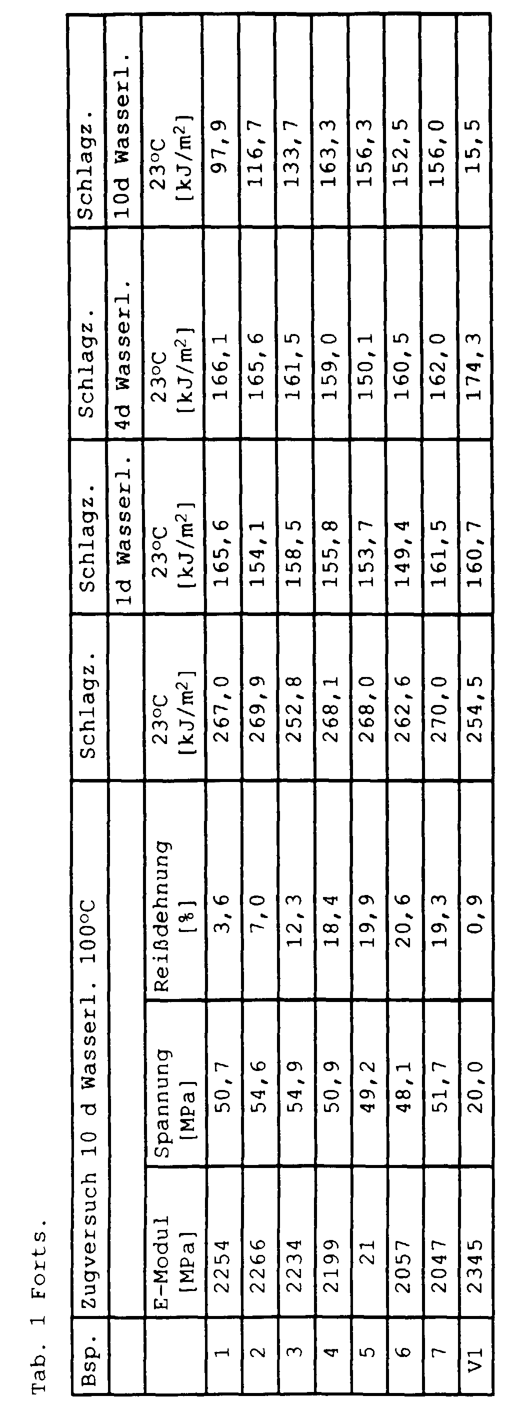

### Beispiele 8 bis 13 und Vergleichsbeispiel 2

Die Komponenten A und B) wurden entsprechend den Bedingungen der Beispiele 1 bis 7 konfektioniert und granuliert. Anschließend wurde das Granulat in fester Phase in einen Taumeltrockner bei 205°C und einer Verweilzeit von 24 Stunden unter Stickstoff auf die in Tabelle 2 angegebenen Viskositätszahlen nachkondensiert (Temperung).

Die Zusammensetzung der Formmassen und die Ergebnisse der Messungen sind der Tabelle 2 zu entnehmen.

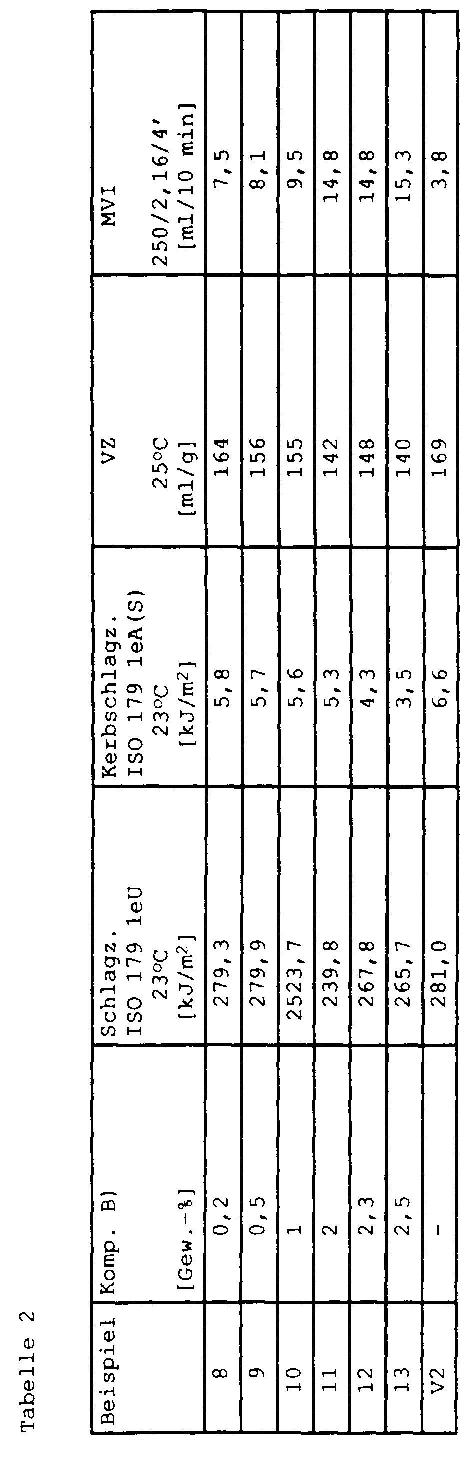

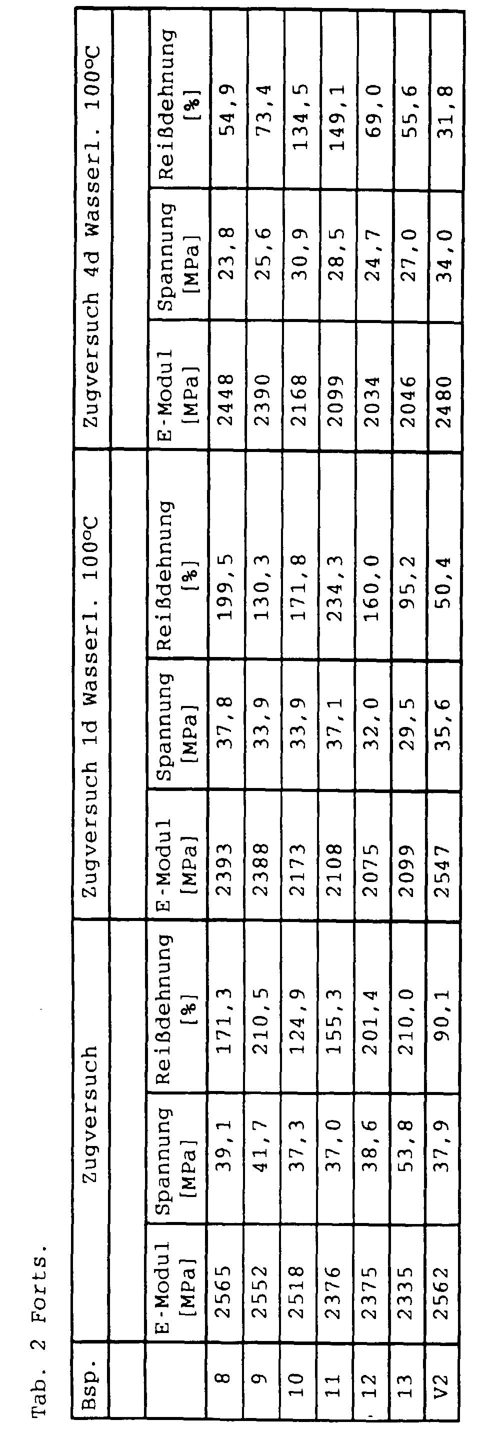

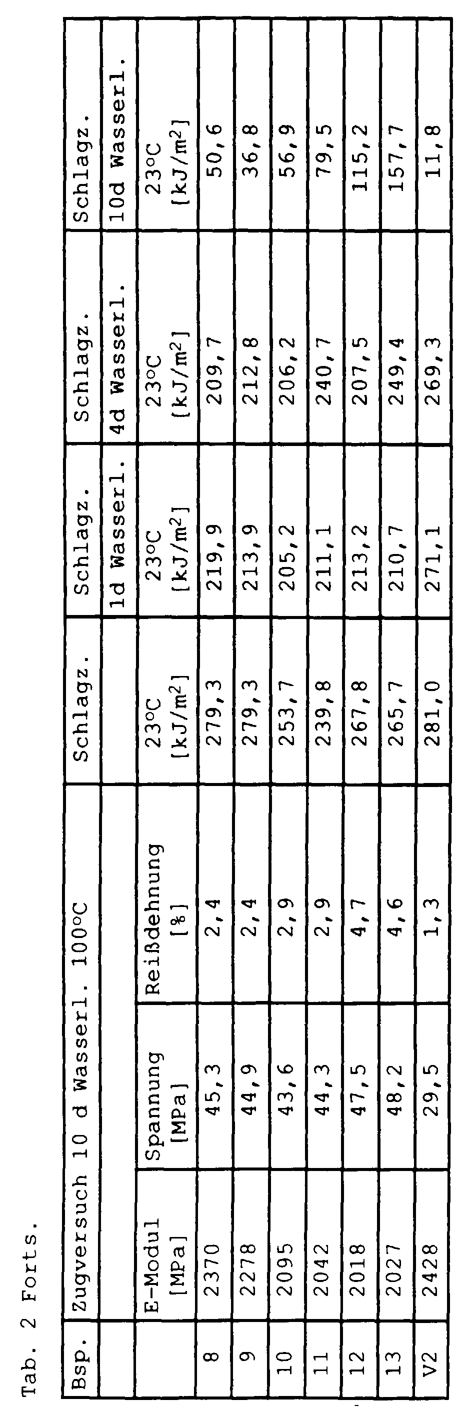

## Patentansprüche

1. Thermoplastische Formmassen, enthaltend
A) 20 - 99 Gew.-% eines Polyalkylenterephthalats mit 2 bis 10 C-Atomen im Alkoholteil, welches gegebenenfalls bis zu 90 Gew.-% bezogen auf A) durch ein Polycarbonat oder ein Polyamid ersetzt sein kann
B) 0,1 - 7 Gew.-% eines Carbodiimids der Formel I 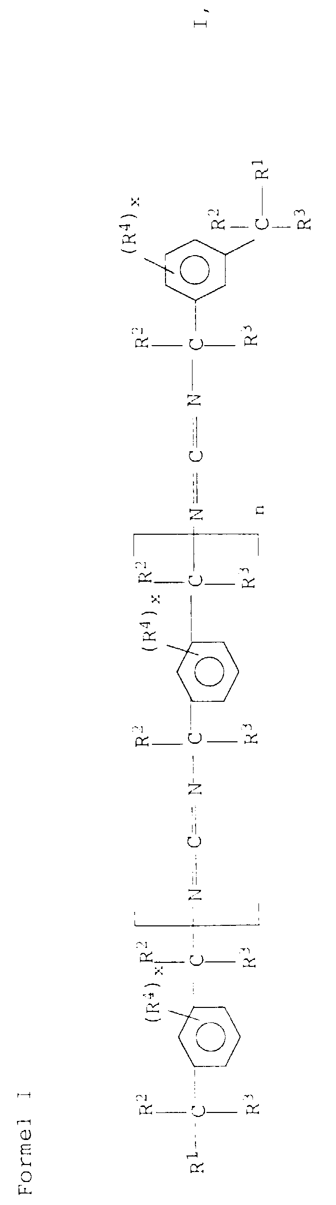 wobei
R¹ gleiche oder verschiedene Reste ausgewählt aus der Gruppe -NCO, -NHCONHR⁵, NHCONR⁵R⁶ und -NHCOOR⁷ bedeutet, wobei
R⁵, R⁶ gleich oder verschieden sind und einen Alkyl- , Cycloalkyl- oder Aralkylrest bedeuten,
R⁷ gleich R⁵ oder ein Alkoxy(poly)oxyalkylenrest ist und
R², R³ gleiche oder verschiedene aliphatische Reste mit 1 bis 18 C-Atomen oder cycloaliphatische Reste mit 5 bis 15 C-Atomen oder aromatische Reste mit 6 bis 15 C-Atomen bedeuten,
R⁴ gleiche oder verschiedene aliphatische Reste mit 2 bis 20 C-Atomen sowie Halogen- oder ein Alkoxyrest
x eine ganze Zahl von 0 bis 4 und
n eine ganze Zahl von 0 bis 10 bedeutet,
C) 0 bis 75 Gew.-% üblicher Zusatzstoffe und Verarbeitungshilfsmittel,
wobei die Gewichtsprozente der Komponenten A) bis C) zusammen 100 % ergeben.

2. Thermoplastische Formmassen nach Anspruch 1, enthaltend
A) 35 - 99,5
B) 0,5 - 5

3. Thermoplastische Formmassen nach der Ansprüchen 1 oder 2, in denen die Komponente B) ein Carbodiimid der Formel II 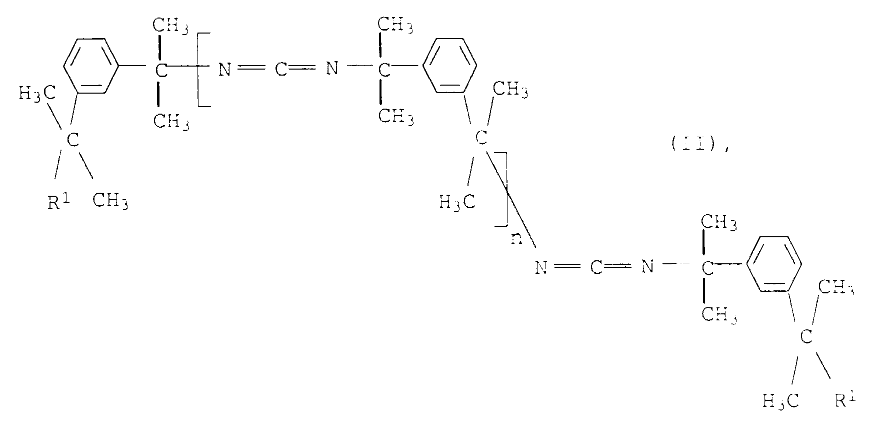 in der
R¹ gleiche oder verschiedene Reste ausgewählt aus der Gruppe -NCO, -NHCONHR⁵, NHCONR⁵R⁶ und -NBCOOR⁷ bedeuten, wobei
R⁵, R⁶ gleich oder verschieden sind und einen Alkyl-, Cycloalkyl- oder Aralkylrest bedeuten und
R⁷ gleich R⁵ oder ein Alkoxy(poly)oxyalkylenrest ist und
n eine ganze Zahl von 0 bis 10 darsnellt.

4. Thermoplastische Formmassen nach den Ansprüchen 1 bis 3, enthaltend als Komponente B) ein Carbodiimid und/oder oligomere Polycarbondiimide der Formel (I), in der R¹ eine -NHCOOR⁷-Gruppe ist und R⁷ einen Alkoxypolyoxyethylenrest mit einem Molekulargewicht von 76 bis 2000 und n eine ganze Zahl von 0 bis 10 bedeuten.

5. Thermoplastische Formmassen nach der Anprüchen 1 bis 4, enthaltend als Komponente B ein Carbodiimid und/oder oligomere Polycarbodiimide der Formel (III), 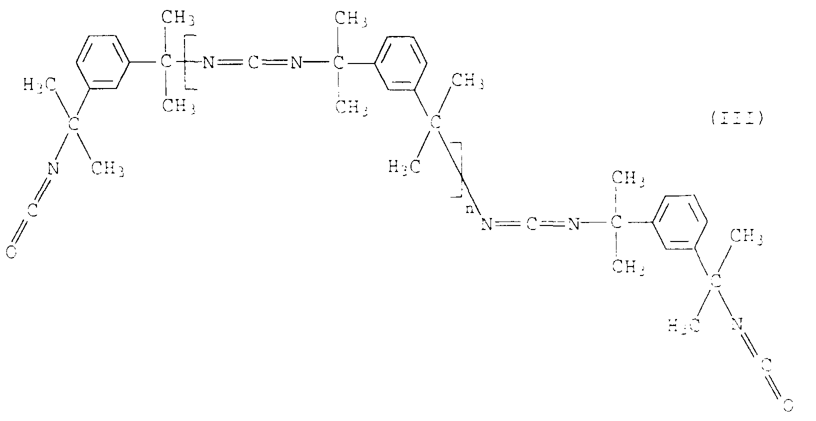 in der n eine ganze Zahl von 0 bis 10 ist.

6. Thermoplastische Formmassen nach Anspruch 3, in denen das Carbodiimid der Formel II erhältlich ist
a) durch Kondensation unter Kohlendioxidabspaltung von 1,3-Bis-(1-methyl-1-isocyanato-ethyl)-benzol und gegebenenfalls teilweise oder vollständige Umsetzung der endständigen Isocyanatgruppen des erhaltenen Carbodiimids oder/und der erhaltenen oligomeren Polycarbodiimide mit mindestens einem aliphatischen, cycloaliphatischen oder araliphatischen Amin, Alkohol und/oder Alkoxypolyoxyalkylenalkohol oder
b) durch Umsetzung von bis zu 50 % der Isocyanatgruppen des 1,3-Bis-(1-methyl-1-isocyanato-ethyl)-benzols mit mindestens einem aliphatischen, cycloaliphatischen oder araliphatischen Amin, Alkohol und/oder Alkoxypolyoxyalkylenalkohol und anschließende Kondensation der freien Isocyanatgruppen unter Kohlendioxidabspaltung.

7. Verwendung der thermoplastischen Formmassen gemäß den Ansprüchen 1 bis 6 zur Herstellung von Fasern, Folien und Formkörpern.

8. Formkörper, erhältlich aus den thermoplastischen Formmassen gemäß den Ansprüchen 1 bis 6.

## Claims

1. A thermoplastic molding composition comprising
A) from 20 to 99% by weight of a polyalkylene terephthalate having from 2 to 10 carbon atoms in the alcohol moiety, up to 90% by weight of which may be replaced by a polycarbonate or a polyamide,
B) from 0.1 to 7% by weight of a carbodiimide of the formula I 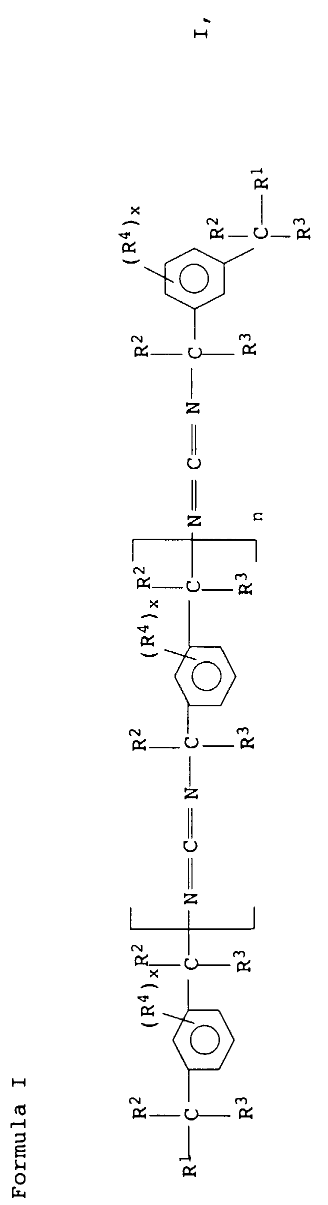 where
R¹ are identical or different radicals selected from the group consisting of -NCO, -NHCONHR⁵, -NHCONR⁵R⁶ and -NHCOOR⁷, where
R⁵, R⁶ are identical or different and are alkyl, cycloalkyl or aralkyl,
R⁷ is the same as R⁵ or is alkoxy(poly)oxyalkylene and
R², R³ are identical or different aliphatic radicals having from 1 to 18 carbon atoms or cycloaliphatic radicals having from 5 to 15 carbon atoms or aromatic radicals having from 6 to 15 carbon atoms,
R⁴ are identical or different aliphatic radicals having from 2 to 20 carbon atoms or halogen or alkoxy,
x is an integer from 0 to 4 and
n is an integer from 0 to 10,
C) from 0 to 75% by weight of conventional additives and processing aids,
where the sum of the percentages by weight of components A) to C) is 100%.

2. A thermoplastic molding composition as claimed in claim 1 comprising
A) from 35 to 99.5
B) from 0.5 to 5

3. A thermoplastic molding composition as claimed in claim 1 or 2 in which component B) is a carbodiimide of the formula II 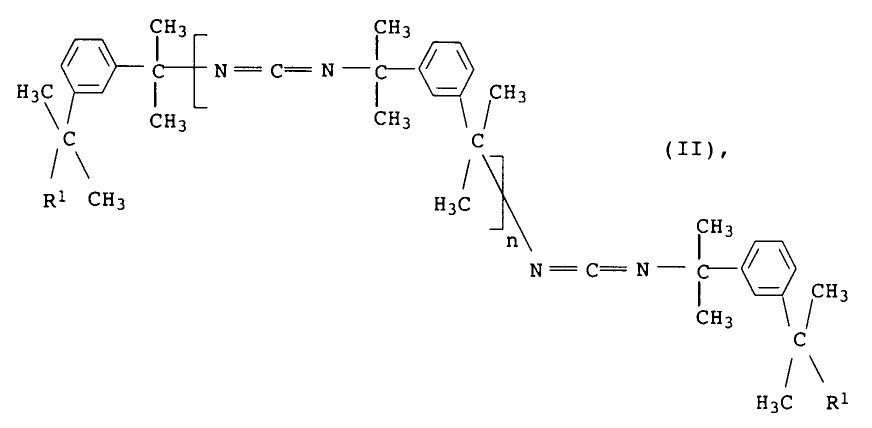 where
R¹ are identical or different radicals selected from the group consisting of -NCO, -NHCONHR⁵, -NHCONR⁵R⁶ and -NHCOOR⁷, where
R⁵, R⁶ are identical or different and are alkyl, cycloalkyl or aralkyl, and
R⁷ is the same as R⁵ or is alkoxy(poly)oxyalkylene and
n is an integer from 0 to 10.

4. A thermoplastic molding composition as claimed in any of claims 1 to 3, containing, as component B), a carbodiimide and/or oligomeric polycarbodiimides of the formula (I), where R¹ is -NHCOOR⁷ and R⁷ is alkoxypolyoxyethylene with a molecular weight of from 76 to 2000 and n is an integer from 0 to 10.

5. A thermoplastic molding composition as claimed in one of claims 1 to 4, containing, as component B), a carbodiimide and/or oligomeric polycarbodiimides of the formula (III), 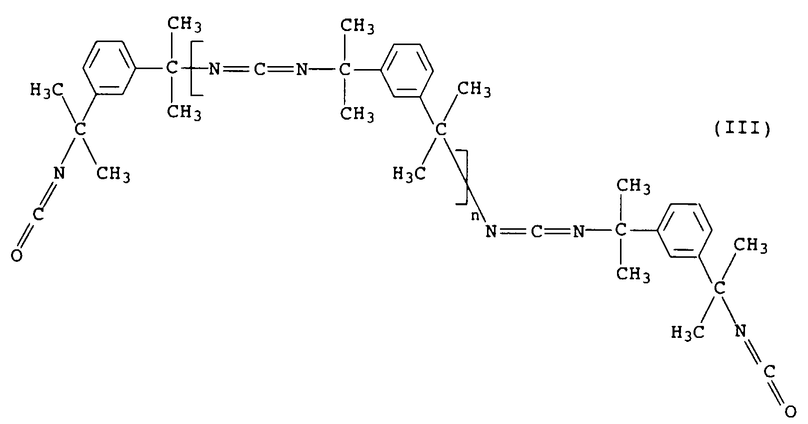 where n is an integer from 0 to 10.

6. A thermoplastic molding composition as claimed in claim 3, in which the carbodiimide of the formula II is obtainable
a) by condensing 1,3-bis(1-methyl-1-isocyanatoethyl)benzene with elimination of carbon dioxide and, if desired, reacting some or all of the terminal isocyanate groups of the resultant carbodiimide and/or oligomeric polycarbodiimides with at least one aliphatic, cycloaliphatic or araliphatic amine, alcohol and/or alkoxypolyoxyalkylene alcohol or
b) by reacting up to 50% of the isocyanate groups of the 1,3-bis(1-methyl-1-isocyanatoethyl)benzene with at least one aliphatic, cycloaliphatic or araliphatic amine, alcohol and/or alkoxypolyoxyalkylene alcohol, and then condensing the free isocyanate groups with elimination of carbon dioxide.

7. A method of using a thermoplastic molding composition as claimed in any of claims 1 to 6 for producing fibers, films or shaped articles.

8. A shaped article obtainable from the thermoplastic molding composition as claimed in any of claims 1 to 6.

## Revendications

1. Masses de moulage thermoplastiques, contenant
A) 20 à 99% en poids d'un polyalkylène-téréphtalate comportant de 2 à 10 atomes de carbone dans sa partie alcool, qui peut éventuellement être remplacé par jusqu'à 90% en poids, par rapport à A), par un polycarbonate ou un polyamide,
B) 0,1 à 7% en poids d'un carbodiimide de la formule I 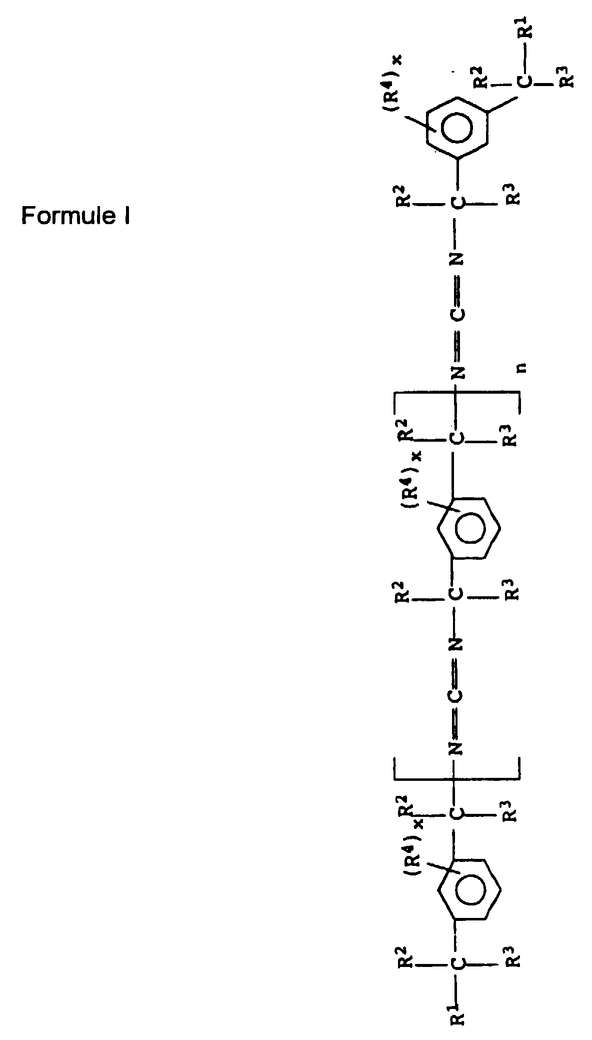 dans laquelle
R¹ sont des restes identiques ou différents choisis dans le groupe formé par -NCO, -NHCONHR⁵, NHCONR⁵R⁶ et -NHCOOR⁷,
où
R⁵, R⁶ sont identiques ou différents et représentent un radical alkyle, cycloalkyle ou aralkyle,
R⁷ est identique à R⁵ ou représente un reste alcoxy(poly)oxyalkylène, et
R², R³ sont identiques ou différents et représentent des restes aliphatiques comportant de 1 à 18 atomes de carbone, ou des restes cycloaliphatiques comportant de 5 à 15 atomes de carbone, ou des restes aromatiques comportant de 6 à 15 atomes de carbone,
R⁴ sont identiques ou différents et représentent des restes aliphatiques comportant de 2 à 20 atomes de carbone, ainsi que des radicaux halogène ou alcoxy,
x est un nombre entier de 0 à 4 et
n est un nombre entier de 0 à 10,
C) 0 à 75% en poids d'autres additifs et adjuvants de traitement,
les pourcentages en poids des composants A) à C) totalisant ensemble 100%.

2. Masses de moulage thermoplastiques selon la revendication 1, contenant
A) 35 à 99,5
B) 0,5 à 5.

3. Masses de moulage thermoplastiques selon les revendications 1 ou 2, caractérisées en ce que le composant B) est un carbodiimide de la formule II 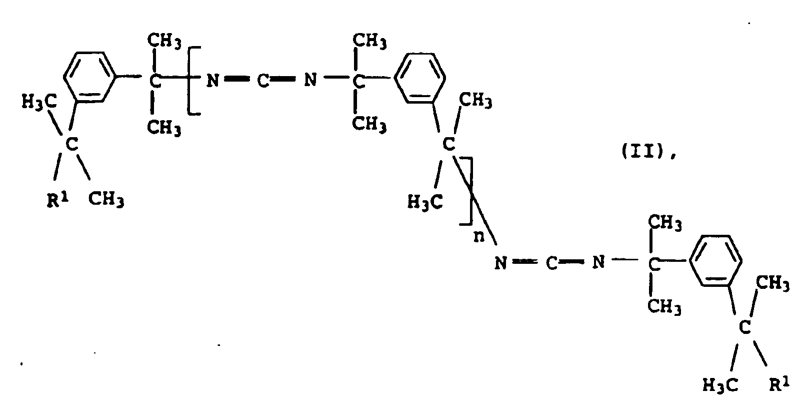 dans laquelle
R¹ sont des restes identiques ou différents choisis dans le groupe formé par -NCO, -NHCONHR⁵, NHCONR⁵R⁶ et -NHCOOR⁷,
où
R⁵, R⁶ sont identiques ou différents et représentent un radical alkyle, cycloalkyle ou aralkyle,
R⁷ est identique à R⁵ ou représente un reste alcoxy(poly)oxyalkylène, et
n est un nombre entier de 0 à 10.

4. Masses de moulage thermoplastiques selon les revendications 1 à 3, contenant en tant que composant B) un carbodiimide et/ou des polycarbodiimides oligomères de la formule (I), dans laquelle R¹ est un groupe -NHCOOR⁷ et R⁷ est un reste alcoxypolyoxyéthylène d'un poids moléculaire de 76 à 2000 et n est un nombre entier de 0 à 10.

5. Masses de moulage thermoplastiques selon les revendications 1 à 4, contenant en tant que composant B un carbodiimide et/ou des polycarbodiimides oligomères de la formule (III) 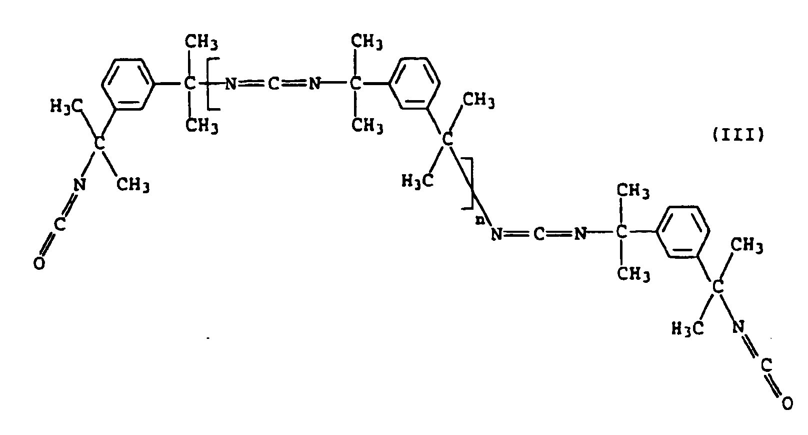 dans laquelle n est un nombre entier de 0 à 10.

6. Masses de moulage thermoplastiques selon la revendication 3, caractérisée en ce que le carbodiimide de la formule II peut être obtenu par:
a) condensation avec élimination de dioxyde de carbone de 1,3-bis(1-méthyl-1-isocyanatoéthyl)benzène et éventuellement réaction partielle ou totale des groupes terminaux d'isocyanate du carbodiimide présent et/ou des polycarbodiimides oligomères présents avec au moins un alcool alcoxypolyoxyalkylénique et/ou un alcool et ou une amine aliphatique, cycloaliphatique ou araliphatique, ou
b) réaction de jusqu'à 50% des groupes isocyanate du 1,3-bis(1-méthyl-1-isocyanatoéthyl)benzène avec au moins un alcool alcoxypolyoxyalkylénique et/ou un alcool et ou une amine aliphatique, cycloaliphatique ou araliphatique et condensation subséquente des groupes isocyanate libre avec élimination de dioxyde de carbone.

7. Utilisation des masses de moulage thermoplastiques selon les revendications 1 à 6 pour la production de fibres, feuilles et articles façonnés.

8. Articles façonnés que l'on peut obtenir à partir des masses de moulages thermoplastiques selon les revendications 1 à 6.
